# EUROPEAN PATENT APPLICATION

(11) **EP 3 045 442 A1**
(43) Date of publication of application: **20.07.2016**
(21) Application number: 14844894.7
(22) Date of filing: 11.09.2014
(51) Int. Cl.: C07C 29/04, C07C 1/207, C07C 13/20, C07C 33/14, C07C 67/14, C07C 69/24

(54) **METHOD FOR PRODUCING CYCLOLAVANDULOL AND DERIVATIVE THEREOF**

(30) Priority: 12.09.2013 JP 2013189554
(71) Applicant: Shin-Etsu Chemical Co., Ltd., Tokyo 100-0004 (JP)
(72) Inventor: KINSHO, Takeshi, Joetsu-shi Niigata 942-8601 (JP); ISHIBASHI, Naoki, Joetsu-shi Niigata 942-8601 (JP)
(74) Representative: Vidon Brevets & Stratégie
(86) International application number: PCT/JP2014/074081
(87) International publication number: WO 2015/037664

(57) **Abstract**

A targeted compound is produced simply, efficiently and selectively. Specifically provided are a method for producing (2,4,4-trimethyl-2-cyclohexene)methanol, comprising the steps of: reacting the carbonyl group of 2,4,4-trimethyl-2-cyclohexenone (1) to obtain 1-methylene-2,4,4-trimethyl-2-cyclohexene (2) and reacting the exocyclic double bond of Compound (2) to obtain the (2,4,4-trimethyl-2-cyclohexene)methanol (3); and a method for producing a (2,4,4-trimethyl-2-cyclohexenyl)methyl ester compound, comprising a step of esterifying Compound (3) to obtain the (2,4,4-trimethyl-2-cyclohexenyl)methyl ester compound (4).

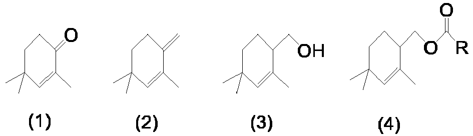

## Description

### Technical Field

The present invention relates to methods for producing a monoterpene alcohol and a derivative thereof that are important as biologically active substances or synthetic intermediates thereof. More specifically, the present invention relates to methods for producing (2,4,4-trimethyl-2-cyclohexene)methanol known as cyclolavandulol and for producing a (2,4,4-trimethyl-2-cyclohexenyl)methyl ester compound derived from the alcohol compound.

### Background Art

The sex pheromones of insects are biologically active substances that are commonly secreted by female individuals and have the function of attracting male individuals. A small amount of the sex pheromone shows strong attractive activities. The sex pheromone has been widely used as means for forecasting insect emergence or for ascertaining regional spread (invasion into a specific area) and as means for controlling an insect pest. As the means for controlling insect pests, control methods called mass trapping, lure and kill (another name: attract and kill), lure and infect (another name: attract and infect), and mating disruption are widely used in practice. To utilize the sex pheromone, economical production of a required amount of the pheromone product is demanded for basic research and also for application.

(2,4,4-Trimethyl-2-cyclohexenyl)methyl butyrate ester (the other names: (2,4,4-trimethyl-2-cyclohexenyl)methyl n-butyrate, cyclolavandulyl butyrate) has been isolated as an attractant of an important wasp in controlling mealybug. More specifically, Tabata et al. have found (2,4,4-trimethyl-2-cyclohexenyl)methyl butyrate, which is a substance attracting Anagyrus sawadai that is one of the wasps living parasitically with the mealybug; have isolated it as the active substance from byproducts generated when lavandulol is reacted with butyryl chloride to form lavandulyl butyrate; and have identified its structure (Non-Patent Document 1). Tabata et al. have also found that lavandulol as the starting material is reacted with butyryl chloride to produce (2,4,4-trimethyl-2-cyclohexenyl)methyl butyrate in a yield of 1.2%.

Teshiba et al. have reported that this substance attracts a wasp, Anagyrus sawadai, which does not intrinsically live parasitically with Planococcus kraunhiae, and another wasp, Leptomastix dactylopii, in persimmon fields where Planococcus kraunhiae damaged the trees; and thus has the possibility of providing effective means for controlling Planococcus kraunhiae (Non-Patent Document 2).

For basic biological and pharmaceutical researches including structure-activity studies of these biologically active substances and also for the purpose of application and practical use, sufficient amounts of the substances are required to be supplied. Accordingly, there is a strong demand for efficient and selective methods for producing these biologically active substances, where the amounts of isomeric byproducts are small and separation can be easily done.

### Prior art documents

### Non-Patent documents

Non-Patent Document 1: Appl. Entomol. Zool., 46, 117-123 (2011)
Non-Patent Document 2: Journal of Pharmacy Research, 4, 2126-2128 (2011)
Non-Patent Document 3: Appl. Entomol. Zool., 43, 369-375 (2008)

### Summary of the Invention

### Problems to be solved by the Invention

However, these synthesis examples involve a long process in a low yield or involve intermediate purification techniques that are difficult to perform on an industrial scale, such as chromatography. Thus, it is considered to be very difficult to supply a product in a required amount.

In view of the above circumstances, an object of the present invention is to provide a simple, efficient and selective production method in order to supply a sufficient amount of product required for biological, pharmacological or agronomical activity studies, practical applications and other purposes.

### Solution to the Problems

As a result of intensive studies for achieving the object, the inventors of the present invention have found that production of (2,4,4-trimethyl-2-cyclohexene)methanol from 2,4,4-trimethyl-2-cyclohexenone as a starting material and further production of a (2,4,4-trimethyl-2-cyclohexenyl)methyl ester compound through esterification of the alcohol can be carried out in high yields with high selectivity on an industrial scale, and have accomplished the present invention.

In an aspect of the present invention, there is provided a method for producing (2,4,4-trimethyl-2-cyclohexene)methanol, comprising the steps of: reacting a carbonyl group of 2,4,4-trimethyl-2-cyclohexenone represented by Formula (1) below to obtain 1-methylene-2,4,4-trimethyl-2-cyclohexene represented by Formula (2) below; and reacting an exocyclic double bond of the 1-methylene-2,4,4-trimethyl-2-cyclohexene to obtain the (2,4,4-trimethyl-2-cyclohexene)methanol represented by Formula (3) below.

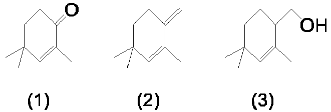

In another aspect of the present invention, there is provides a method for producing a (2,4,4-trimethyl-2-cyclohexenyl)methyl ester compound, comprising: the steps comprised by the above method to obtain (2,4,4-trimethyl-2-cyclohexene)methanol; and a step of esterifying the (2,4,4-trimethyl-2-cyclohexene)methanol to obtain the (2,4,4-trimethyl-2-cyclohexenyl)methyl ester compound represented by General Formula (4): 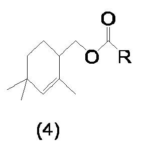 where R represents a hydrogen atom or a monovalent hydrocarbon group having 1 to 10 carbon atoms.

### Effect of the Invention

According to the present invention, a monoterpene alcohol and a derivative thereof, which are important as biologically active substances or synthetic intermediates thereof, that is, (2,4,4-trimethyl-2-cyclohexene)methanol and (2,4,4-trimethyl-2-cyclohexenyl)methyl ester compounds can be synthesized simply, efficiently and selectively.

### Modes for Carrying out the Invention

Embodiments of the present invention will now be described in detail, but the present invention is not limited to them.

According to the invention, the starting material, 2,4,4-trimethyl-2-cyclohexenone (1) can be easily synthesized, for example, from an enamine which is an aldehyde derivative, and ethyl vinyl ketone by the enamine method (G. Stork et al., Journal of Organic Chemistry, 85, 207-221; and Y. Chan et al., Organic Syntheses, Coll. Vol. 6, 496-498). Subsequent one-carbon (C1) homologation and functional group transformation of the starting material can produce respective target compounds.

The first step is a step of converting the carbonyl group of 2,4,4-trimethyl-2-cyclohexenone (1) as the starting material to a methylene group, thereby forming 1-methylene-2,4,4-trimethyl-2-cyclohexene (2).

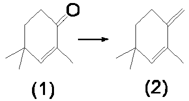

In the step, a method selected from various types of known methods such as the Wittig reaction using a phosphorus ylide, the Peterson reaction involving addition of an α-silylmethyl carbanion and subsequent acid or base treatment, a method of using a Tebbe complex, a method of using a Petasis reagent, a method of using a reagent obtained by treatment of diiodomethane or dibromomethane with zinc in the presence of titanium tetrachloride, and a method involving addition of a methyl carbanion (a methyl organometallic reagent) such as a methylmagnesium halide, methyllithium and trimethylaluminum and subsequent dehydration of the resulting tertiary alcohol can be used. Among them, it is preferable to use the Wittig reaction or the Peterson reaction from the standpoint of obtaining a single isomer without change of the position of an existing double bond. It is particularly preferable to use the Wittig reaction from the standpoint of reaction conditions and ease in post-treatment and product isolation.

In a preferable example of the Wittig reaction, triphenylphosphonium methylide [(C₆H₅)₃P=CH₂], which is a phosphorus ylide reagent prepared by treating a triphenylmethylphosphonium halide with a base in a solvent, is reacted with the ketone as the starting material.

Examples of the triphenylmethylphosphonium halide to be used in the preparation of the phosphorus ylide reagent include triphenylmethylphosphonium chloride, triphenylmethylphosphonium bromide, and triphenylmethylphosphonium iodide.

Examples of the solvent to be used in the preparation of the phosphorus ylide reagent include ethers such as diethyl ether, di-n-butyl ether, tetrahydrofuran and 1,4-dioxane; hydrocarbons such as hexane, heptane, benzene, toluene, xylene and cumene; aprotic polar solvents such as N,N-dimethylformamide (DMF), 1,3-dimethyl-2-imidazolidinone (DMI), dimethyl sulfoxide (DMSO) and hexamethylphosphoric triamide (HMPA); and nitriles such as acetonitrile and propionitrile. The solvent can be used singly or in combination of two or more.

Examples of the base to be used in the preparation of the phosphorus ylide reagent include organometallic reagents such as methyllithium, ethyllithium, n-butyllithium and methylmagnesium chloride; alkoxides such as sodium methoxide, sodium ethoxide and potassium t-butoxide; metal amides such as lithium diisopropylamide, lithium hexamethyldisilazide, sodium hexamethyldisilazide and lithium dicyclohexylamide; metal hydrides such as sodium hydride, potassium hydride and calcium hydride; and dimsyl sodium (sodium methylsulfinylmethylide). The amount of the base is preferably 0.5 to 2 mol, more preferably 1.0 to 1.5 mol relative to 1 mol of the triphenylmethylphosphonium halide.

The reaction temperature in the preparation of the phosphorus ylide reagent is preferably -78 to 50°C, more preferably -78°C to room temperature (i.e. 5 to 35°C, the same applies hereinafter), even more preferably -10°C to room temperature.

The reaction time in the preparation of the phosphorus ylide reagent is preferably 5 minutes to 18 hours. It is more preferably 5 minutes to 1 hour from the standpoint of reagent stability.

Triphenylphosphonium methylide prepared in this manner, which is a phosphorus ylide reagent, is reacted with the ketone 2,4,4-trimethyl-2-cyclohexenone (1). Typically, the ketone without solvent or the ketone diluted with a solvent is added dropwise to a solution of the phosphorus ylide reagent.

The solvent to be used for the dilution can include the same examples as those of the solvent used in the preparation of the phosphorus ylide reagent.

The reaction temperature of the Wittig reaction is preferably -78 to 50°C, more preferably -78°C to room temperature, even more preferably -10°C to room temperature.

The amount of the phosphorus ylide reagent to be used for the Wittig reaction is preferably 0.5 to 10 mol relative to 1 mol of the ketone as the reactant. It is more preferably 1.0 to 2.5 mol relative to 1 mol of the ketone from the viewpoint of yield and cost efficiency.

The reaction time of the Wittig reaction may be the time sufficient to complete the reaction, which may be determined by monitoring the progress of the reaction through gas chromatography (GC) or thin-layer chromatography (TLC). The reaction time of the Wittig reaction is typically 30 minutes to 96 hours.

The post-treatment of the Wittig reaction, which is the isolation and purification of the target compound, can be carried out by using a method appropriately selected from purification methods commonly used in organic syntheses, such as vacuum distillation and various types of chromatography. The vacuum distillation is preferable from the standpoint of industrial cost efficiency. In this case, triphenylphosphine oxide generated by the reaction can be precipitated with a poor solvent and removed by filtration or the like by advance. Alternatively, the reaction mixture can be directly distilled under reduced pressure without removal of triphenylphosphine oxide. As a result, 1-methylene-2,4,4-trimethyl-2-cyclohexene (2) is obtained. When the target compound has sufficient purity, the product without purification can be directly subjected to the subsequent step.

The next step is a step of converting 1-methylene-2,4,4-trimethyl-2-cyclohexene (2) into (2,4,4-trimethyl-2-cyclohexene)methanol (3).

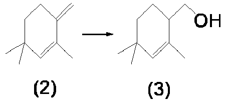

In the step, one of various types of known methods of hydrolyzing an olefin can be used. It is preferably a method by hydroboration-oxidation, or a method of hydrosilylation and subsequent conversion of a silicon substituent into a hydroxy group in the presence of fluorine ion. The method by hydroboration-oxidation is particularly preferable from the standpoint of the regioselective introduction of a hydroxy group through selection of a reagent.

In the hydroboration, the reactant 1-methylene-2,4,4-trimethyl-2-cyclohexene (2) is typically reacted with a boron compound (borane) in a solvent.

Examples of the boron compound to be used include unsubstituted boranes (e.g. BH₃, the dimer thereof and complexes with ethers and amines) and substituted boranes such as monoalkyl boranes and dialkyl boranes. The target compound (2,4,4-trimethyl-2-cyclohexene)methanol (3) is a primary alcohol having a hydroxyl group introduced to an exo-side of the methylene group at the 1-position of the reactant 1-methylene-2,4,4-trimethyl-2-cyclohexene (2). It is thus desired that the internal olefin at the 2-position of the reactant be not reacted but only the methylene group at the 1-position be reacted without generation of a tertiary alcohol 1,2,4,4-tetramethyl-2-cyclohexen-1-ol as a by-product. To achieve the desired selectivity, the boron compound is preferably a monoalkyl borane or a dialkyl borane. A borane with large steric hindrance is particularly preferred and includes thexylborane, isopinocampheylborane, dicyclohexylborane, disiamylborane, diisopinocampheylborane and 9-borabicyclo[3.3.1]nonane (9-BBN). As described later, an alcohol compound derived from the alkyl substituent of a substituted borane is inevitably formed in the subsequent oxidation step, and thus the borane is preferably selected in consideration of the separation from the target compound. Such a boron compound can be separately prepared or can be a commercially available. The boron compound can be prepared in the system and reacted with the reactant in situ.

The amount of the boron compound to be used for the hydroboration is preferably 0.5 to 30 mol relative to 1 mol of the reactant 1-methylene-2,4,4-trimethyl-2-cyclohexene (2). It is more preferably 1.0 to 10 mol relative to 1 mol of the reactant (2) from the standpoint of yield, cost efficiency and selectivity.

The reaction solvent for the hydroboration is preferably an ether such as diethyl ether, di-n-butyl ether, tetrahydrofuran and 1,4-dioxane. The ether can be mixed with one or more solvents selected from the group consisting of hydrocarbons such as hexane, heptane, benzene, toluene, xylene and cumene; aprotic polar solvents such as N,N-dimethylformamide (DMF), 1,3-dimethyl-2-imidazolidinone (DMI), dimethyl sulfoxide (DMSO) and hexamethylphosphoric triamide (HMPA); and nitriles such as acetonitrile and propionitrile.

The reaction temperature of the hydroboration is, for example, -78°C to the boiling point of a solvent and can be selected in consideration of reactivity, selectivity and reaction rate.

The reaction time of the hydroboration may be preferably the time sufficient to complete the reaction, which may be determined by monitoring the progress of the reaction through thin-layer chromatography (TLC) or the like, and is typically 30 minutes to 96 hours.

The subsequent oxidation step is a step of subjecting the substituted borane obtained by the hydroboration to oxidation treatment with an alkaline hydrogen peroxide to obtain a target alcohol compound (2,4,4-trimethyl-2-cyclohexene)methanol (3). This step typically comprises dropwise addition of an alkaline solution to the reaction mixture of hydroboration and the subsequent dropwise addition of an aqueous hydrogen peroxide solution thereto. In order to suppress a sudden increase in temperature of the reaction mixture during the dropwise additions, the solutions are slowly added dropwise to the reaction mixture cooled at preferably from -20°C to icecooling.

As the alkaline solution in the oxidation step, an aqueous sodium hydroxide solution is typically used. As the hydrogen peroxide in the oxidation step, a commercially available 35% aqueous solution is preferably used and may be appropriately diluted for use. Since the alkaline solution and the hydrogen peroxide are available at low cost in large amounts on an industrial scale, the amounts of the alkaline solution and the hydrogen peroxide can be any amounts that are required to complete the reaction.

The isolation and purification of the target compound can be carried out by a method appropriately selected from purification methods commonly used in organic syntheses, such as vacuum distillation and various types of chromatography. The vacuum distillation is preferable from the standpoint of industrial cost efficiency. When a substituted borane is used for the hydroboration, the alcohol compound derived from an alkyl group of the substituted borane can be separated from the target compound by solvent removal or vacuum distillation.

As explained above, (2,4,4-trimethyl-2-cyclohexene)methanol (3) can be obtained from 2,4,4-trimethyl-2-cyclohexenone (1) in a high yield with high selectivity.

The obtained (2,4,4-trimethyl-2-cyclohexene)methanol (3) can be converted into a (2,4,4-trimethyl-2-cyclohexenyl)methyl ester (4) through esterification.

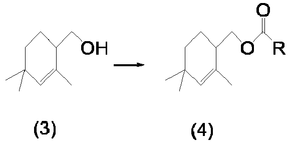

R represents a hydrogen atom or a monovalent hydrocarbon group having 1 to 10 carbon atoms, preferably 1 to 5 carbon atoms. Depending on the type of R, the ester compound can have a different structure. For example, when R is a hydrogen atom, the ester compound is a formate.

Specific examples of the monovalent hydrocarbon group of R include linear, branched or cyclic saturated monovalent hydrocarbon groups such as a methyl group (an acetate as the ester compound), an ethyl group (a propionate as the ester compound), an n-propyl group, an isopropyl group, an n-butyl group, an isobutyl group, a sec-butyl group, a tert-butyl group, an n-pentyl group, an isopentyl group, an n-hexyl group, an n-octyl group, an n-nonyl group, an n-decyl group, a cyclopropyl group, a cyclobutyl group, a cyclopentyl group, a cyclohexyl group, a methylcyclopropyl group, dimethylcyclopropyl groups (including all regioisomers with respect to the positions of methyl groups, the same applies hereinafter), methylcyclobutyl groups, dimethylcyclobutyl groups, trimethylcyclobutyl groups, tetramethylcyclobutyl groups, methylcyclopentyl groups, dimethylcyclopentyl groups, trimethylcyclopentyl groups, tetramethylcyclopentyl groups, methylcyclohexyl groups, dimethylcyclohexyl groups and trimethylcyclohexyl groups; linear, branched or cyclic unsaturated hydrocarbon groups such as a vinyl group (an acrylate as the ester compound), a 1-propenyl group (a crotonate as the ester compound), a 2-propenyl group (a methacrylate as the ester compound), a 2-methyl-1-propenyl group (a senecioate as the ester compound), an ethynyl group (a propiolate as the ester compound), a propynyl group, a 1-butyny group, cyclopentenyl groups (including all regioisomers with respect to the position of a double bond, the same applies hereinafter), cyclohexenyl groups, dicyclohexadienyl groups and methylcyclohexenyl groups; and monovalent hydrocarbon groups that are isomeric with these groups.

For the esterification, a known ester production method such as a reaction with an acylating agent, a reaction with a carboxylic acid or a transesterification can be employed.

When the reaction with an acylating agent is carried out as the esterification, the solvent is preferably selected from chlorinated solvents such as methylene chloride, chloroform and trichloroethylene; hydrocarbons such as hexane, heptane, benzene, toluene, xylene and cumene; ethers such as diethyl ether, dibutyl ether, diethylene glycol diethyl ether, diethylene glycol dimethyl ether, tetrahydrofuran and 1,4-dioxane; nitriles such as acetonitrile; ketones such as acetone and 2-butanone; esters such as ethyl acetate and n-butyl acetate; and aprotic polar solvents such as N,N-dimethylformamide, dimethyl sulfoxide and hexamethylphosphoric triamide. The solvent can be used singly or in combination of two or more.

The acylating agent is preferably an acid halide or an acid anhydride including mixed acid anhydrides. Examples of the acid halide preferably include acid chlorides (RCOC1 wherein R corresponds to a monovalent hydrocarbon group of R in Formula (4)) and acid bromides (RCOBr wherein R corresponds to R in Formula (4)). Examples of the acid anhydride including mixed acid anhydrides preferably include RCOOX wherein R corresponds R in Formula (4) and X represents a leaving group such as R²C=O wherein R² represents a hydrogen atom or a monovalent hydrocarbon group having 1 to 10 carbon atoms, preferably 1 to 5 carbon atoms, may be the same as or different from R, preferably the same as R, and includes the same examples as those for R. Examples of leaving group X further include a trifluoroacetyl group, a methanesulfonyl group, a trifluoromethanesulfonyl group, a benzenesulfonyl group, a p-toluenesulfonyl group and a p-nitrophenyl group.

In the reaction with the acylating agent, the reactant (2,4,4-trimethyl-2-cyclohexene)methanol (3), the acylating agent and a base such as triethylamine, diisopropylethylamine, N,N-dimethylaniline, pyridine and 4-dimethylaminopyridine are sequentially or simultaneously added to the solvent and reacted. In the reaction with an acylating agent such as acid anhydrides, the reaction can be carried out in the presence of an acid catalyst selected from inorganic acids such as hydrochloric acid, hydrobromic acid, sulfuric acid and nitric acid and organic acids such as oxalic acid, trifluoroacetic acid, methanesulfonic acid, benzenesulfonic acid and p-toluenesulfonic acid, instead of the base.

The amount of the acylating agent depends on the structure of the reactant and is preferably in a range of 1 to 40 mol, more preferably 1 to 5 mol relative to 1 mol of the alcohol compound as the reactant.

The reaction temperature of the acylation can be selected appropriately in accordance with the type of an acylating agent to be used and reaction conditions. It is in general preferably -50°C to the boiling point of a solvent, more preferably -20°C to room temperature.

When the reaction with a carboxylic acid is carried out as the esterification, the reaction is a dehydration reaction between the carboxylic acid and the reactant alcohol compound (2,4,4-trimethyl-2-cyclohexene)methanol (3), typically in the presence of an acid catalyst.

The amount of the carboxylic acid depends on the structure of the reactant and is preferably in a range of 1 to 40 mol, more preferably 1 to 5 mol relative to 1 mol of the reactant alcohol compound.

Examples of the acid catalyst to be used for the reaction with a carboxylic acid include inorganic acids such as hydrochloric acid, hydrobromic acid, sulfuric acid and nitric acid; and organic acids such as oxalic acid, trifluoroacetic acid, methanesulfonic acid, benzenesulfonic acid and p-toluenesulfonic acid. The acid catalyst is used singly or in combination of two or more. The amount of the acid catalyst is preferably 0.001 to 1 mol, more preferably a catalytic amount of 0.01 to 0.05 mol relative to 1 mol of the reactant alcohol compound.

The solvent to be used in the reaction with a carboxylic acid can include the same examples as those of the solvent to be used in the reaction with an acylating agent. The reaction temperature is in general preferably -50°C to the boiling point of the solvent. The reaction may be carried out while removing generated water from the system by azeotropy, making use of a solvent including hydrocarbons such as hexane, heptane, benzene, toluene, xylene and cumene. In this case, the water can be distilled off while the reaction mixture is refluxed at the boiling point of a solvent at normal pressure. Alternatively, the water can be distilled off at a temperature lower than the boiling point under reduced pressure.

When the transesterification is carried out as the esterification, the reactant alcohol compound is reacted with a carboxylate ester compound produced from a corresponding carboxylic acid and a lower alcohol in the presence of a catalyst, while removing the resulting lower alcohol.

The carboxylate ester compound is preferably a primary alkyl ester and is particularly preferably a methyl ester, an ethyl ester or an n-propyl ester from the standpoint of price and ease in progress of the reaction. The amount of the carboxylate ester compound depends on the structure of the reactant and is preferably in a range of 1 to 40 mol, more preferably 1 to 5 mol relative to 1 mol of the reactant alcohol compound.

Examples of the catalyst to be used for the transesterification include inorganic acids such as hydrochloric acid, hydrobromic acid, sulfuric acid and nitric acid; organic acids such as oxalic acid, trifluoroacetic acid, methanesulfonic acid, benzenesulfonic acid and p-toluenesulfonic acid; bases such as sodium methoxide, sodium ethoxide, potassium t-butoxide and 4-dimethylaminopyridine; salts such as sodium cyanide, potassium cyanide, sodium acetate, potassium acetate, calcium acetate, tin acetate, aluminum acetate, aluminum acetoacetate and alumina; and Lewis acids such as aluminum trichloride, aluminum ethoxide, aluminum isopropoxide, boron trifluoride, boron trichloride, boron tribromide, tin tetrachloride, tin tetrabromide, dibutyltin dichloride, dibutyltin dimethoxide, dibutyltin oxide, titanium tetrachloride, titanium tetrabromide, titanium(IV) methoxide, titanium(IV) ethoxide, titanium(IV) isopropoxide and titanium(IV) oxide. The catalyst is used singly or in combination of two or more.

The amount of the catalyst to be used for the transesterification is preferably 0.001 to 20 mol, more preferably a catalytic amount of 0.01 to 0.05 mol relative to 1 mol of the reactant alcohol compound. The reaction can be carried out without a solvent (the carboxylate ester as the reaction reagent may also serve as the solvent). The solvent-free reaction is preferable because of unnecessity of additional operations such as concentration and solvent recovery. In order to prevent the target compound or a reaction reagent from polymerizing or for other purposes, a solvent can be used supplementarily.

Examples of the solvent to be used for the transesterification include hydrocarbons such as hexane, heptane, benzene, toluene, xylene and cumene; and ethers such as diethyl ether, dibutyl ether, diethylene glycol diethyl ether, diethylene glycol dimethyl ether, tetrahydrofuran and 1,4-dioxane. The solvent can be used singly or in combination of two or more.

The reaction temperature of the transesterification can be selected appropriately in accordance with the type of a carboxylate ester compound to be used and reaction conditions. The reaction is typically carried out with heating. The reaction is preferably carried out at around the boiling point of lower alcohol having a low boiling point and being generated by the transesterification, while distilling off the generated lower alcohol, so as to obtain better results. The lower alcohol includes methanol, ethanol and 1-propanol. The alcohol may be distilled off under reduced pressure at a temperature lower than the boiling point.

The isolation and purification of the target (2,4,4-trimethyl-2-cyclohexenyl)methyl ester compound (4) can be carried out by a method appropriately selected from purification methods commonly used in organic syntheses, such as vacuum distillation and various types of chromatography. The vacuum distillation is preferable from the standpoint of industrial cost efficiency.

As explained above, the (2,4,4-trimethyl-2-cyclohexenyl)methyl ester compound (4) can be obtained from 2,4,4-trimethyl-2-cyclohexenone (1) in a high yield with high selectivity.

### EXAMPLES

The present invention will next be described in further detail with reference to Examples. It should not be construed that the present invention is limited to or by them.

### <Synthesis Example 1 >

### Synthesis of Starting Material 2,4,4-Trimethyl-2-Cyclohexenone (1)

The starting material 2,4,4-trimethyl-2-cyclohexenone (1) was synthesized through the following reaction route, specifically by the following method.

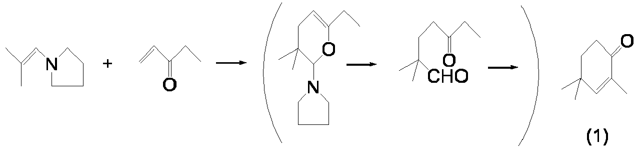

Under a nitrogen atmosphere, 40.4 g of ethyl vinyl ketone was added dropwise to 68.5 g of ice-cooled isobutyraldehyde pyrrolidine enamine over 10 minutes. After the dropwise addition, the temperature of the reaction mixture was gradually increased to room temperature, and the reaction mixture was further stirred at room temperature for 17 hours. The reaction mixture was re-cooled on ice, and then 400 ml of 20% hydrochloric acid was added dropwise thereto. After the dropwise addition, the temperature of the reaction mixture was gradually increased to room temperature, and the reaction mixture was further stirred at room temperature for 30 hours. The reaction mixture was extracted with diethyl ether, then the diethyl ether phase was separated, and the aqueous phase was neutralized with sodium hydrogen carbonate and then was further extracted with diethyl ether. The combined diethyl ether phase was dried over sodium sulfate and concentrated under reduced pressure. The obtained crude product was distilled under reduced pressure to obtain 53.9 g of target compound (yield 82%).

### 2,4,4-Trimethyl-2-cyclohexenone (1)

### Colorless liquid

Boiling point: 76°C/1.9 kPa
IR (D-ATR): v=2958, 2925, 2867, 1676, 1448, 1362 cm⁻¹.
EI-MS (70 eV): m/z=27, 41, 55, 67, 81, 95, 110, 123, 138 (M⁺).
¹H-NMR (500 MHz, CDCl₃): δ=1.11 (6H, s), 1.70 (3H, d, J=1.5 Hz), 1.81 (2H, dt-like, J=0.8, 6.9 Hz), 2.42 (2H, t-like, J=7.0 Hz), 6.37-6.39 (1H, m) ppm.
¹³C-NMR (125 MHz, CDCl₃): δ=15.91, 27.93 (2C), 32.89, 34.44, 36.33, 132.47, 155.07, 199.73 ppm.

### <Example 1>

### Synthesis of 1-Methylene-2,4,4-Trimethyl-2-Cyclohexene (2)

Under a nitrogen atmosphere, 55.0 g of 2,4,4-trimethyl-2-cyclohexenone (1) synthesized by the method in Synthesis Example 1 was added dropwise over 20 minutes to an ice-cooled phosphorus ylide solution containing 181 g of methyltriphenylphosphonium bromide and 56.1 g of potassium t-butoxide in a mixed solvent of 700 ml of tetrahydrofuran and 300 ml of toluene. The mixture was stirred for 80 minutes still on ice and then poured into ice water. The organic phase was separated, and the aqueous phase was extracted with diethyl ether. The combined organic phase was washed with a saturated sodium chloride solution, then dried over magnesium sulfate, and concentrated under reduced pressure. N-hexane was added to the residue, and the resulting triphenylphosphine oxide was filtered off. The filtrate was concentrated under reduced pressure, and the obtained crude product was distilled under reduced pressure to obtain 44.31 g of target compound (yield 83%).

### 1-Methylene-2,4,4-trimethyl-2-cyclohexene (2)

### Colorless liquid

Boiling point: 110°C/21 kPa
EI-MS (70 eV): m/z=27, 39, 53, 65, 79, 93, 105, 121, 136 (M⁺).
IR (D-ATR): v=2955, 2922, 2854, 1604, 1440, 878 cm⁻¹.
¹H-NMR (500 MHz, CDCl₃): δ=1.01 (6H, s), 1.50-1.53 (3H, m), 1.79 (2H, d, J=1.5 Hz), 2.37-2.41 (2H, m), 4.77 (1H, quint-like, J=1.5 Hz), 4.87 (1H, br. s), 5.39 (1H, s) ppm.
¹³C-NMR (125 MHz, CDCl₃): δ=19.65, 28.97, 29.20 (2C), 32.66, 37.44, 107.58, 129.95, 138.59, 144.50 ppm.

### <Example 2>

### Synthesis Example 1 of (2,4,4-trimethyl-2-cyclohexene)methanol (3)

Under a nitrogen atmosphere, 19.0 g of 2-methyl-2-butene was added dropwise to 145 ml of ice-cooled 0.9 M borane-tetrahydrofuran solution over 5 minutes, and the mixture was stirred on ice for 2 hours to prepare disiamylborane in the system.

Next, a mixture of 11.24 g of 1-methylene-2,4,4-trimethyl-2-cyclohexene (2) synthesized by the method in Example 1 and 50 ml of tetrahydrofuran was added dropwise to the prepared disiamylborane over 5 minutes. The reaction mixture was stirred on ice for 5 minutes, then the ice bath was removed, and the mixture was stirred overnight at room temperature.

The reaction mixture was re-cooled on ice; then 12 ml of 99.5% by weight ethanol, 44 g of 25% aqueous sodium hydroxide solution, and 44 g of 35% aqueous hydrogen peroxide were carefully, sequentially added thereto; and the mixture was stirred on ice for 30 minutes and further stirred at room temperature for 1 hour. The reaction mixture was subjected to addition of 25 ml of water, then the organic phase was separated, and the aqueous phase was extracted with ether. The combined organic phase was washed with a saturated sodium chloride solution, then dried over magnesium sulfate, and concentrated under reduced pressure to obtain 19.87 g of crude product (gas chromatography purity of 76.0% and yield calculated on basis of purity: 84%).

In various chromatographic and spectral analyses of the crude product, neither an isomer (2,4,4-trimethyl-1-cyclohexene)methanol nor an isomer 1,2,4,4-tetramethyl-2-cyclohexen-1-ol was detected, and the endocyclic double bond of the cyclohexene ring did not move. The results indicated that the hydroboration had proceeded selectively from the exo-side. The crude product had sufficient purity as the intermediate and was used without purification in the subsequent step.

### (2,4,4-Trimethyl-2-cyclohexene)methanol (3)

### Colorless liquid

EI-MS (70 eV): m/z=31, 41, 55, 67, 81, 93, 108, 123, 139, 154 (M⁺).
¹H-NMR (500 MHz, CDCl₃): δ=0.937 (3H, s), 0.941 (3H, s), 1.32-1.40 (2H, m), 1.45-1.52 (1H, m), 1.65-1.68 (3H, m), 1.68-1.74 (2H, m), 2.50-2.12 (1H, m), 3.56-3.71 (2H, m), 5.28 (1H, s) ppm.

### <Example 3>

### Synthesis Example 2 of (2,4,4-Trimethyl-2-Cyclohexene)Methanol (3)

The target compound (2,4,4-trimethyl-2-cyclohexene)methanol (3) was prepared in a quantitative yield from 1-methylene-2,4,4-trimethyl-2-cyclohexene (2) by a method in accordance with that in Example 2 except that 9-borabicyclo[3.3.1]nonane (9-BBN) was used in the place of the disiamylborane prepared in the system in Example 2. Various spectra of the obtained target compound were identical with those of the target compound in Example 2.

### <Example 4>

### Synthesis of (2,4,4-Trimethyl-2-Cyclohexenyl)Methyl Butyrate Corresponding to n-Propyl Group of R in Formula (4)

Under a nitrogen atmosphere, 18.9 g of butyryl chloride was added dropwise to an ice-cooled mixture of 18.8 g of (2,4,4-trimethyl-2-cyclohexene)methanol (3) synthesized by the method in Example 2, 14.5 g of pyridine and 200 ml of acetonitrile over 10 minutes. The mixture was stirred on ice for 10 minutes and stirred at room temperature for 90 minutes. The reaction mixture was then poured into ice water and extracted with n-hexane. The separated organic phase was washed with a diluted hydrochloric acid, water, a saturated aqueous sodium hydrogen carbonate solution, and a saturated sodium chloride solution, then was dried over magnesium sulfate, and concentrated under reduced pressure. The obtained residue was distilled under reduced pressure to obtain 17.42 g of target compound (yield 84%).

### (2,4,4-trimethyl-2-cyclohexenyl)methyl butyrate

### Colorless liquid

Boiling point: 80-83°C/400 Pa
EI-MS (70 eV): m/z=27, 43, 55, 71, 93, 108, 121, 136.
IR (D-ATR): v=2956, 2934, 2864, 1738, 1453, 1303, 1176 cm⁻¹.
¹H-NMR (500 MHz, CDCl₃): δ=0.91-0.97 (9H, m), 1.29-1.36 (1H, m), 1.41-1.48 (1H, m), 1.56-1.73 (7H, m), 2.17-2.23 (1H, m), 2.28 (2H, t, J=7.2 Hz), 3.98 (1H, dd, J=8.4, 11 Hz), 4.14 (1H, dd, J=4.0, 11 Hz), 5.23 (1H, s) ppm.
¹³C-NMR (150 MHz, CDCl₃): δ=13.67, 18.44, 22.03, 22.83, 29.56, 30.06, 31.78, 33.76, 36.29, 38.30, 65.43, 130.11, 135.79, 173.81 ppm.

These spectral data were in good agreement with those in Non-Patent Document 1.

## Claims

1. A method for producing (2,4,4-trimethyl-2-cyclohexene)methanol, comprising the steps of:
reacting a carbonyl group of 2,4,4-trimethyl-2-cyclohexenone represented by Formula (1): 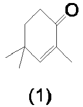 to obtain 1-methylene-2,4,4-trimethyl-2-cyclohexene represented by Formula (2): 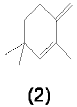 and,
reacting an exocyclic double bond of the obtained 1-methylene-2,4,4-trimethyl-2-cyclohexene to obtain the (2,4,4-trimethyl-2-cyclohexene)methanol represented by Formula (3):

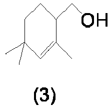

2. A method for producing a (2,4,4-trimethyl-2-cyclohexenyl)methyl ester compound, comprising:
the steps comprised by the method according to claim 1 to obtain (2,4,4-trimethyl-2-cyclohexene)methanol, and
a step of esterifying the (2,4,4-trimethyl-2-cyclohexene)methanol to obtain the (2,4,4-trimethyl-2-cyclohexenyl)methyl ester compound represented by General Formula (4): 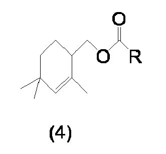 wherein R represents a hydrogen atom or a monovalent hydrocarbon group having 1 to 10 carbon atoms.
